(19) Europäisches Patentamt
European Patent Office
Office européen des brevets

(11) Publication number: **0 206 471 B1**

(12) **EUROPEAN PATENT SPECIFICATION**

(45) Date of publication of patent specification: **14.10.92**

(51) Int. Cl.[5]: **C12P 17/18**, C07D 471/04

(21) Application number: **86303110.0**

(22) Date of filing: **24.04.86**

(54) **Process for preparation of pyrrolo-quinoline quinone.**

(30) Priority: **24.04.85 JP 88255/85**
**18.07.85 JP 159238/85**

(43) Date of publication of application:
**30.12.86 Bulletin 86/52**

(45) Publication of the grant of the patent:
**14.10.92 Bulletin 92/42**

(84) Designated Contracting States:
**DE FR GB NL**

(56) References cited:
**EP-A- 0 164 943**

**CHEMICAL ABSTRACTS, vol. 100, no. 21, 21st May 1984, page 496, abstract no. 173083r, Columbus, Ohio, US; M. AMEYAMA et al.: "Microbial production of pyrroloquinoline quinone", & AGRIC. BIOL. CHEM. 1984, 48(2), 561-5**

**CHEMICAL ABSTRACTS, vol. 103, no. 9, 2nd September 1985, page 358, abstract no. 68030k, Columbus, Ohio, US; M. SHIMAO et al.: "Enhancement of pyrroloquinoline quinone production and poly(vinyl alcohol) degradation in mixed continuous cultures of Pseudomonas putida VM15A and Pseudomonas sp, strain VM15C with mixed carbon sources", & APPL. ENVIRON. MICROBIOL. 1985, 49(6), 1389-91**

**PATENT ABSTRACTS OF JAPAN, vol. 8, no. 232 (C-248), 25th October 1984**

(73) Proprietor: **MITSUBISHI GAS CHEMICAL COMPANY, INC.**
**5-2, Marunouchi 2-chome Chiyoda-Ku**
**Tokyo, 100(JP)**

(72) Inventor: **Urakami, Teizi**
**25-25, Taihei 3-chome**
**Niigata-shi Niigata 950(JP)**

(74) Representative: **Sheard, Andrew Gregory et al**
**Kilburn & Strode 30, John Street**
**London WC1N 2DD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to a process for preparing pyrrolo-quinoline quinone, more particularly a process for microbiologically preparing the same by use of bacteria.

Pyrrolo-quinoline quinone (hereinafter abbreviated to "PQQ") is 2,7,9-tricarboxy-1H-pyrrolo[2,3-f]-quinoline-4,5-dione having the formula

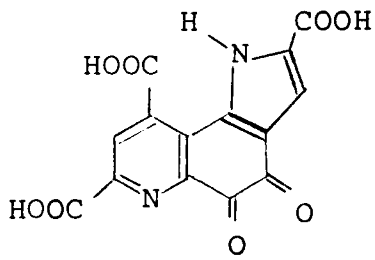

PQQ is purified and crystallized as a coenzyme of methanol dehydrogenase produced by methanol-utilizing bacteria (S.A. Salisbury et al., Nature, Vol. 280, pp. 843-844, 1979). In recent years, PQQ has also been found in such eucaryotes as fungi and yeasts and mammals, as well as in bacteria. PQQ is considered to play an important role in a medical field, since it is a coenzyme which facilitates enzymatic reactions or metabolism.

One of processes for producing PQQ is organic chemical synthesis (JACS. Vol. 103, pp. 5599-5600, 1981). However, the organic chemical synthesis is not economical, because the synthesis needs so many steps that it takes a very long time until it is completed and it requires troublesome steps for removal of isomers and various other by-products. Furthermore, the yield of PQQ is not so high. Another process is a microbiological one (Japanese published unexamined patent application 59-113896). However, this process is not economical either, because the yield is as low as 0.1 - 0.3 x $10^{-8}$ mole/ℓ (hereinafter referred to as "M"), i.e., 4 - 12 µg per litre.

The present inventor has been studying how to obtain PQQ by use of bacteria. The inventor has found that the bacteria given below are capable of producing a large amount of PQQ: the genera Achromobacter, Methylobacillus, Methylomonas, Methanomonas, Protaminobacter, Methylobacterium, Protomonas, Mycoplana, Ancylobacter, Microcyclus, Hyphomicrobium, Xanthobacter, Thiobacillus, Alteromonas or Methylophaga, and a certain species belonging to genus Pseudomonas.

According to a first aspect of the present invention, a processs for producing PQQ is provided wherein the bacterium defined below is cultured in a medium containing, as a carbon source, a source of methanol and/or a source of methylamine, and then PQQ is recovered from a culture broth or a supernatant of the culture broth, said bacterium having the capability of utilizing methanol and/or methylamine to produce PQQ and being selected from the genus Achromobacter, Methylobacillus, Methylomonas, Methanomonas, Protaminobacter, Methylobacterium, Protomonas, Mycoplana, Ancylobacter, Microcyclus, Hyphomicrobium, Xanthobacter, Thiobacillus, Alteromonas , Methylophaga or a certain species belonging to genus Pseudomonas.

EP-A-0164943 also relates to the production of PQQ by culturing bacteria. However, this document contains no reference to the bacteria used in the present invention other than some of the genus Protaminobacter. Ameyama et al ((1984) Agric. Biol. Chem 48 (2) 561-565) also disclose PQQ prepared by culturing Pseudomonas M5 and Pseudomonas AMI. The use of other bacteria in this method is not disclosed.

Any bacterium may be employed as long as it has capability of utilizing methanol and/or methylamine to produce a large amount of PQQ and belongs to genus Achromobacter, Methylobacillus, Methylomonas, Methanomonas, Protaminobacter, Methylobacterium, Protomonas, Mycoplana, Ancylobacter, Microcyclus, Hyphomicrobium, Xanthobacter, Thiobacillus, Alteromonas or Methylophaga, or a certain species of genus Pseudomonas.

According to a second aspect of the invention there is provided a process for the preparation of pyrrolo-quinoline quinone which comprises culturing a bacterium as defined below in a medium containing as a carbon source a source of methylamine and recovering pyrrolo-quinoline quinone thus produced from a culture broth or a supernatant of the culture broth, wherein the bacterium possesses the capability of utilizing methylamine to produce pyrrolo-quinoline quinone and belongs to the species Pseudomonas

aminovorans.

Examples of suitable bacteria for use in the methods of the invention are Achromobacter methanophila ATCC 21275 (= JCM 2841, ditto ATCC 21452, ditto ATCC 21961 (= JCM 2843), Methylobacillus glycogenes ATCC 29475 (= JCM 2850 = NCIB 11375), Methylomonas methanolica NRRL B-5458 (= JCM 2851), Methylomonas thalassica ATCC 33146, Methylomonas clara ATCC 31226 (= NCIB 11809), Methanomonas methylovora ATCC 21852 (= JCM 2840 = NCIB 11376), ditto ATCC 21369 (= JCM 2844), ditto ATCC 21958 (= JCM 2847), ditto ATCC 21963 (= JCM 2848), Methanomonas methylovora subsp. thianimophila ATCC 21370 (= JCM 2849), Protaminobacter candidus ATCC 21372 (= JCM 2852), ditto ATCC 21959, Protaminobacter thiaminophagus ATCC 21371 (= JCM 2853), ditto ATCC 21926, ditto ATCC 21927, ditto ATCC 21957, ditto ATCC 21969, Pseudomonas aminovorans NCIB 9039, Methylobacterium organophilum ATCC 29983 (= NCIB 11278), Protomonas extorquens JCM 2802 (= DSM 1337 = NCIB 9399), ditto JCM 2805 (= NCIB 9133 = ATCC 14718 = DSM 1338), ditto JCM 2806 (= DSM 1339 = NCIB 9686), ditto JCM 2811 (= ATCC 14821), ditto JCM 2812 (= NCIB 10598), ditto JCM 2813 (= NCIB 10599), ditto JCM 2814 (= NCIB 10600), ditto JCM 2815 (= NCIB 10602), ditto JCM 2816 (= NCIB 10611), ditto JCM 2817 (= NCIB 10601), ditto JCM 2818 (= NCIB 10603), ditto JCM 2819 (= NCIB 10606), ditto JCM 2820 (= NCIB 10607), ditto JCM 2821 (= NCIB 10608), ditto JCM 2822 (= NCIB 10609), ditto JCM 2823 (= NCIB 10610), ditto JCM 2824 (= NCIB 10612), ditto JCM 2825 (= NCIB 10604), ditto JCM 2826 (= NCIB 10605), ditto JCM 2827 (= ATCC 21438), ditto JCM 2829 (= ATCC 29983 = ICPB 4095), ditto JCM 2830 (= ATCC 27329 = IAM 12099), ditto JCM 2831 (= IAM 12098), ditto JCM 2832 (names of Protomonas extorquens are based on International Journal of Systematic Bacteriology, Vol. 34, pp. 188-201, (1984)), Mycoplana rubra NCIB 10409 (= JCM 2803), Ancylobacter aquaticus ATCC 25396 (= CCM 1786 = DSM 101 = NCIB 9721), ditto DSM 334, ditto ATCC 27068, ditto ATCC 27069 (names of Ancylobacter aquaticus are based on International Journal of Systematic Bacteriology, Vol. 33, pp. 397-398, (1983)), Microcyclus eburneus ATCC 21373 (= DSM 1106), Mycrocyclus polymorphum NCIB 10516 (= DSM 2457), Microcyclus methanolica DSM 2666, ditto DSM 2667, ditto DSM 2668, ditto DSM 2669, Hyphomicrobium variable NCIB 10517, Hyphomicrobium vulgare NCIB 9698, ditto NCIB 9775, Hyphomicrobium methylovorum IFO 14180, Hyphomicrobium sp. DSM 1869, Xanthobacter autotrophicus DSM 432, ditto DSM 431, ditto DSM 597, ditto DSM 685, ditto DSM 1393, ditto DSM 1618, ditto DSM 2009, Xanthobacter flavus NCIB 10071 (= DSM 338), Thiobacillus novellus ATCC 8093 (= CCM 1077 = DSM 506 = IFO 12443 = NCIB 9113), ditto NCIB 10456, Thiobacillus versutus ATCC 25364 (names of Thiobacillus versutus are based on International Journal of Systematic Bacteriology, Vol. 33, pp. 211-217, (1983)), Alteromonas thalassomethanolica ATCC 33145, Methylophaga marina NCMB 2244 (= ATCC 35842), Methylophaga thalassica NCMB 2162, and Methylophaga thalassica NCMB 2163.

Variants obtained from the above strains may also be employed.

The medium employed has to contain, as a carbon source, a source of methanol and/or a source of methylamine ($CH_3NH_2$). Methylamine hydrochloride may be used as a source for methylamine. Concentrations of methanol and/or methylamine in the medium vary depending on bacteria employed. They are usually 3 wt.% or less, preferably 1.5 wt.% or less for methanol and 1 wt.% or less, preferably 0.5 wt.% or less for methylamine, on the basis of the medium, from a practial point of view. The medium further will generally contain proper amounts of usual nitrogen sources and inorganic and/or organic salts. Ammonium sulphate, urea, ammonium nitrate, ammonium phosphate, peptone, meat extract, etc. may be used as the nitrogen source. Phosphates, magnesium salts, iron salts, and, if necessary, a small amount of the other metal salts are used as the inorganic and/or organic salts. Furthermore, amino acid, nucleic acid, vitamin, yeast extract, malt extract and other growth promoting materials may be added. If the bacteria essentially require a nutrient, the medium has to contain necessary materials to this effect. Two to four wt.% of NaCl is added to the medium when the bacterium employed is Methylomonas thalassica ATCC 33146 or Alteromonas thalassomethanolica ATCC 33145, since these bacteria require NaCl for their growth. Instead, sea water may be used in place of NaCl and water for preparing the medium. The presence of $Na^+$ and/or $Mg^{++}$ is essential in the medium for genus Methylophaga, since all of the strains thereof mentioned above require $Na^+$ and $Mg^{++}$ for their growth. A preferable source for $Na^+$ is sea water, since the strains belonging to Methylophaga are marine bacteria. Instead, NaCl may be added to the medium until NaCl concentration is 2 - 4 %.

Culturing conditions vary depending upon bacteria employed. Also usually necessary, from a practical viewpoint, is to select a culture temperature and pH of the culture broth within the usual ranges of from 25°C to 45°C and of from 6 to 8, respectively, taking into account the growth and propagation of bacteria employed. When the nitrogen source is an ammonium salt, in particular, the pH of the medium becomes more acidic as the bacteria propagate. Controlling the pH of the culture broth sometimes has to be done by adding alkaline materials, for example, ammonia, caustic potash (potassium hydroxide) or caustic soda

(sodium hydroxide), in order to keep it at a constant level during the culturing. Ammonia is preferred. Culturing may be effected batchwise or continuously.

PQQ produced after the culturing is recoverable by any means familiar to those skilled in the art. For instance, solid-liquid separation, for example, filtration and centrifugation, may be applied to the culture broth in order to obtain supernatant by separation of cells from a culture broth. The supernatant and culture broth as it is before, for example, the filtration, which contains cells, may be used for the recovery. Recovery from the supernatant or the culture broth is effected, for example, by ion-exchange chromatography, gel-filtration of concentrate, solvent extraction of a dried product or affinity chromatography.

Identification of PQQ thus recovered is made by, for example, paper chromatography, thin-layer chromatography, elementary analysis, nuclear magnetic resonance spectrometry mass spectrometry, UV absorption spectroscopy and/or high-performance liquid chromatography.

Quantitative analysis may be made by use of a D-glucose dehydrogenase-activity deletion variant of Pseudomonas aeruginosa (Ameyama et al., FEBS Letters, Vol. 130, pp. 179-183, 1981) and E. coli - (Ameyama et al., Agric. Biol. Chem., 49, pp. 1227-1231, 1985), absorption spectra of UV (Dekker et al., European Journal of Biochemistry Vol. 125, pp. 69-73 (1982)), or high-performance liquid chromatography.

The present invention is illustrated by the following examples.

## Example 1

An aqueous solution (200 ml, pH 7.1), which had been prepared by dissolving 3 g of $(NH_4)_2SO_4$, 1.4 g of $KH_2PO_4$, 2.1 g of $Na_2HPO_4$, 0.2 g of $MgSO_4 \cdot 7H_2O$, 30 mg of $CaCl_2 \cdot 2H_2O$, 30 mg of $FeC_6H_5O_7 \cdot XH_2O$, 5 mg of $MnCl_2 \cdot 4H_2O$, 5 mg of $ZnSO_4 \cdot 7H_2O$, 0.5 mg of $CuSO_4 \cdot 5H_2O$, 4 mg of thiamine hydrochloride, 4 mg of calcium pantothenate, 20 µg of biotin and 8 ml of methanol in 1 ℓ of water, was poured into Erlenmeyer flasks (1 ℓ each) and sterilized at 120 °C for 20 minutes. They were designated the main culturing media (hereinafter referred to as the main medium).

Pre-culturing was done separately by culturing each of the bacteria given below in the same main medium as above at 30 °C for 24 hours. The pre-cultured liquids were respectively inoculated in an amount of 1 vol.% to the main media. The liquids thus inoculated were subjected to culturing under the action of rotary shakers at 30 °C. Methanol concentrations in all of the culture broths were lowered to not more than 0.001 vol.% by two days cultivation. The supernatants were obtained by centrifugation of the culture broths, respectively. Amounts of PQQ in the supernatants are shown in Table 1.

Table 1

| Strains | Amount of PQQ (µg/ℓ of supernatant) |
|---|---|
| Achromobacter methanophila ATCC 21275 | 73 |
| Methylobacillus glycogenes ATCC 29475 | 115 |
| Methylomonas clara ATCC 31226 | 160 |
| Protaminobacter candidus ATCC 21372 | 70 |
| Protaminobacter thiaminophagus ATCC 21371 | 80 |
| Pseudomonas methanolica ATCC 21704 | 140 |
| Pseudomonas methylotropha NCIB 10510 | 360 |

| | |
|---|---|
| Methylobacterium organophilum ATCC 29983 | 500 |
| Protomonas extorquens JCM 2802 | 750 |
| Mycoplana rubra NCIB 10409 | 600 |
| Ancylobacter aquaticus ATCC 25396 | 1000 |
| Microcyclus eburneus ATCC 21373 | 800 |
| Microcyclus polymorphum NCIB 10516 | 500 |
| Microcyclus methanolica DSM 2666 | 600 |
| Hyphomicrobium variable NCIB 10517 | 310 |
| Hyphomicrobium vulgare NCIB 9698 | 350 |
| Hyphomicrobium methylovorum IFO 14180 | 400 |
| Hyphomicrobium sp. DSM 1869 | 7000 |
| Xanthobacter autotrophicus DSM 432 | 1200 |
| Xanthobacter flavus NCIB 10071 | 1000 |
| Thiobacillus novellus NCIB 10456 | 2200 |
| Methanomonas methylovora ATCC 21852 | 400 |
| Methanomonas methylovora subsp. thiaminophila ATCC 21370 | 420 |
| Pseudomonas insueta ATCC 21276 | 340 |

Example 2

An aqueous solution (200 mℓ, pH 7.1), which had been prepared by dissolving 3 g of $(NH_4)_2SO_4$, 1.4 g of $KH_2PO_4$, 2.1 g of $Na_2HPO_4$, 0.2 g of $MgSO_4 \cdot 7H_2O$, 30 mg of $CaCl_2 \cdot 2H_2O$, 30 mg of $FeC_6H_5O_7 \cdot XH_2O$, 5 mg of $MnCl_2 \cdot 4H_2O$, 5 mg of $ZnSO_4 \cdot 7H_2O$, 0.5 mg of $CuSO_4 \cdot 5H_2O$ and 5 g of methylamine hydrochloride in 1 ℓ of water, was poured into Erlenmeyer flasks (1 ℓ each) and sterilized at 120 °C for 20 minutes. They were designated main culturing media.

Pre-culturing was separately done by culturing each of Thiobacillus versutus ATCC 25364 and Pseudomonas aminovorans NCIB 9039 in the same main media as above at 30 °C for 24 hours. The pre-cultured liquids thus obtained were respectively inoculated in an amount of 1 vol.% to the main media. The liquids thus obtained were subjected to culturing under the action of rotary shakers at 30 °C. Methylamine concentrations in all of the culture broths were lowered to not more than 0.01 vol.% by two days cultivation. The supernatants were obtained by centrifugation of the culture broths, respectively. Amounts of PQQ in the supernatants were 340 µg and 300 µg per litre, respectively.

Example 3

An aqueous solution (200 ml, pH 7.1), which had been prepared by dissolving 3 g of $(NH_4)_2SO_4$, 1.4 g of $KH_2PO_4$, 2.1 g of $Na_2HPO_4$, 0.2 g of $MgSO_4 \cdot 7H_2O$, 30 mg of $CaCl_2 \cdot 2H_2O$, 30 mg of $FeC_6H_5O_7 \cdot XH_2O$, 5 mg of $MnCl_2 \cdot 4H_2O$, 5 mg of $ZnSO_4 \cdot 7H_2O$, 0.5 mg of $CuSO_4 \cdot 5H_2O$, 0.2 g of yeast extract, 10 µg of vitamin $B_{12}$, and 8 ml of methanol in 1 ℓ of sea water was poured into Erlenmeyer flasks (1 ℓ each) and sterilized at 120 °C for 20 minutes. They were designated main culturing media.

Pre-culturing was done separately by culturing each of the bacteria given below in the same main media as above at 30 °C for 24 hours. The pre-cultured liquids thus obtained were each inoculated in an amount of 1 vol.% to the main media. The liquids thus inoculated were subjected to culturing by rotary shakers at 30 °C. Methanol concentrations in all of the culture broths were lowered to not more than 0.001 vol.% by two days cultivation. The supernatants were obtained by centrifugation of the culture broths,

respectively. Amounts of PQQ therein are shown in Table 2.

Table 2

| Strains | Amount of PQQ (μg/ℓ of supernatant) |
|---|---|
| *Methylomonas thalassica* ATCC 33146 | 2600 |
| *Alteromonas thalassomethanolica* ATCC 33145 | 6600 |

Example 4

An aqueous solution (200 ml, pH 7.1), which had been prepared by dissolving 3 g of $(NH_4)_2SO_4$, 1.4 g of $KH_2PO_4$, 2.1 g of $Na_2HPO_4$, 0.2 g of $MgSO_4 \cdot 7H_2O$, 30 mg of $CaCl_2 \cdot 2H_2O$, 30 mg of $FeC_6H_5O_7 \cdot XH_2O$, 5 mg of $MnCl_2 \cdot 4H_2O$, 5 mg of $ZnSO_4 \cdot 7H_2O$, 0.5 mg of $CuSO_4 \cdot 5H_2O$, 0.2 g of yeast extract, 10 μg of vitamin $B_{12}$ and 8 ml of methanol in 1 ℓ of sea water, was poured into Erlenmeyer flasks (1 ℓ each) and sterilized at 120 °C for 20 minutes. They were designated main culturing media.

Pre-culturing was due separately by culturing each of the bacteria given below each in the same medium as above at 30 °C for 24 hours. The pre-cultured liquids were each inoculated in an amount of 1 vol.% to the main mediums. The liquids thus inoculated were subjected to culturing under the action of rotary shakers at 30°C. Methanol concentrations in the culture broths were lowered to not more than 0.001 vol.% by one and a half days cultivation. The supernatants were obtained by centrifugation of the culture broths, respectively. Amounts of PQQ in the supernatants are shown in Table 3.

Table 3

| Strains | Amount of PQQ (μg/ℓ of supernatant) |
|---|---|
| *Methylophaga marina* NCMB 2244 | 4400 |
| *Methylophaga thalassica* NCMB 2162 | 4000 |
| *Methylophaga thalassica* NCMB 2163 | 2600 |

Example 5

Example 4 was repeated except that one litre of an aqueous NaCl solution containing 30 g of NaCl was used in place of the sea water. Methanol concentrations in all of the culture broths were lowered to not more than 0.001 vol.% by one and a half days cultivation. Amounts of PQQ are given in Table 4.

Table 4

| Strains | Amount of PQQ (μg/ℓ of supernatant) |
| --- | --- |
| *Methylophaga marina* NCMB 2244 | 5000 |
| *Methylophaga thalassica* NCMB 2162 | 4600 |
| *Methylophaga thalassica* NCMB 2163 | 3000 |

Example 6

Example 4 was repeated except that 5 g of methylamine hydrochloride per litre dissolved in sea water was used in place of the methanol. Methylamine concentrations in all of the culture broths were lowered to 0.01 vol.% at the end of the culturing. Amounts of PQQ are given in Table 5.

Table 5

| Strains | Amount of PQQ (μg/ℓ of supernatant) |
| --- | --- |
| *Methylophaga marina* NCMB 2244 | 4200 |
| *Methylophaga thalassica* NCMB 2162 | 4100 |
| *Methylophaga thalassica* NCMB 2163 | 2800 |

Example 7

Example 5 was repeated except that 5 g of methylamine hydrochloride per litre of the medium was used in place of the methanol. Methylamine concentrations in all of the culture broths were lowered to 0.01 vol.% at the end of the culturing. Amounts of PQQ are given in Table 6.

Table 6

| Strains | Amount of PQQ (μg/ℓ of supernatant) |
|---|---|
| Methylophaga marina NCMB 2244 | 4100 |
| Methylophaga thalassica NCMB 2162 | 4300 |
| Methylophaga thalassica NCMB 2163 | 3000 |

According to the present invention, stable PQQ may be obtained at low cost, as disclosed above, by the use of bacteria.

**Claims**
**Claims for the following Contracting States : DE, FR, GB**

1. A process for the preparation of pyrrolo-quinoline quinone which comprises culturing a bacterium as defined below in a medium containing as a carbon source a source of methanol and/or a source of methylamine and recovering pyrrolo-quinoline quinone thus produced from a culture broth or a supernatant of the culture broth& wherein bacterium possesses the capability of utilizing methanol and/or methylamine to produce pyrrolo-quinoline quinone and belongs to the genus Achromobacter, Methylobacillus, Methylomonas, Methanomonas, Protaminobacter (provided that this includes only P. candidus and P. thiaminophagus), Methylobacterium, Protomonas, Mycoplana, Ancylobacter, Microcyclus, Hyphomicrobium, Xanthobacter, Thiobacillus, Alteromonas or Methylophaga.

2. A process as claimed in Claim 1 wherein the bacterium is Achromobacter methanophila, Methylobacillus glycogenes, Methylomonas methanolica. Methylomonas thalassica, Methylomonas clara, Methanomonas methylovora, Methanomonas methylovora subsp. thiaminophila, Protaminobacter candidus or Protaminobacter thiaminophagus, Methylobacterium organophilum, Protomonas extorquens, Mycoplana rubra, Ancylobacter aquaticus, Microcyclus eburneus, Microcyclus polymorphum, Microcyclus methanolica. Hyphomicrobium variable, Hyphomicrobium vulgare, Hyphomicrobium methylovorum, Hyphomicrobium sp., Xanthobacter autotrophicus, Xanthobacter flavus, Thiobacillus novellus, Thiobacillus versutus, Alteromonas thalassomethanolica, Methylophaga marina, or Methylophaga thalassica.

3. A process as claimed in Claim 1, wherein the bacterium is Achromobacter methanophila ATCC 21275, ATCC 21452 or ATCC 21961; Methylobacillus glycogenes ATCC 29475; Methylomonas methanolica NRRL B-5458; Methylomonas thalassica ATCC 33146; Methylomonas clara ATCC 31226; Methanomonas methylovora ATCC 21852, ATCC 21369, ATCC 21958 or ATCC 21963; Methanomonas methylovora subsp. thiaminophila ATCC 21370; Protaminobacter candidus ATCC 21372 or ATCC 21959; Protaminobacter thiaminophagus ATCC 21371, ATCC 21926, ATCC 21927, ATCC 21957 or ATCC 21969; Methylobacterium organophilum ATCC 29983; Protomonas extorquens JCM 2802, JCM 2805, JCM 2806, JCM 2811, JCM 2812, JCM 2813, JCM 2814, JCM 2815, JCM 2816, JCM 2817, JCM 2818, JCM 2819, JCM 2820, JCM 2821, JCM 2822, JCM 2823, JCM 2824, JCM 2825, JCM 2826, JCM 2827, JCM 2829, JCM 2830, JCM 2831, or JCM 2832; Mycoplana rubra NCIB 10409; Ancylobacter aquaticus ATCC 25396, DSM 334, ATCC 27068 or ATCC 27069; Microcyclus eburneus ATCC 21373; Microcyclus polymorphum NCIB 10516; Microcyclus methanolica DSM 2666, DSM 2667, DSM 2668 or DSM 2669; Hyphomicrobium variable NCIB 10517; Hyphomicrobium vulgare NCIB 9698 or NCIB 9775; Hyphomicrobium methylovorum IFO 14180; Hyphomicrobium sp. DSM 1869; Xanthobacter autotrophicus DSM 432, DSM 431, DSM 597, DSM 685, DSM 1393, DSM 1618, or DSM 2009; Xanthobacter flavus NCIB 10071; Thiobacillus novellus NCIB 10456 or ATCC 8093; or Thiobacillus versutus ATCC 25364; Alteromonas thalassomethanolica ATCC 33145; Methylophaga marina NCMB 2244, or Methylophaga thalassica NCMB 2162 or NCMB 2163.

4. A process for the preparation of pyrrolo-quinoline quinone which comprises culturing a bacterium as defined below in a medium containing as a carbon source a source of methylamine and recovering pyrrolo-quinoline quinone thus produced from a culture broth or a supernatant of the culture broth, wherein the bacterium possesses the capability of utilizing methylamine to produce pyrrolo-quinoline quinone and belongs to the species Pseudomonas aminovorans.

5. A process as claimed in Claim 4, wherein the bacterium is Pseudomonas aminovorans NCIB 9039.

6. A process as claimed in any one of Claims 1 to 3, wherein the concentration of methanol in the culture broth is not more than 3 wt.%.

7. A process as claimed in any one of Claims 1 to 3, wherein the concentration of methanol in the culture broth is not more than 1.5 wt.%.

8. A process as claimed in any one of Claims 1 to 7, wherein the concentration of methylamine in the culture broth is not more than 1 wt.%.

9. A process as claimed in any one of Claims 1 to 7, wherein the concentration of methylamine in the culture broth is not more than 0.5 wt.%.

**Claims for the following Contracting State : NL**

1. A process for the preparation of pyrrolo-quinoline quinone which comprises culturing a bacterium as defined below in a medium containing as a carbon source a source of methanol and/or a source of methylamine and recovering pyrrolo-quinoline quinone thus produced from a culture broth or a supernatant of the culture broth, wherein bacterium possesses the capability of utilizing methanol and/or methylamine to produce pyrrolo-quinoline quinone and belongs to the genus Achromobacter, Methylobacillus, Methylomonas, Methanomonas, Protaminobacter, Methylobacterium, Protomonas, Mycoplana, Ancylobacter, Microcyclus, Hyphomicrobium, Xanthobacter, Thiobacillus, Alteromonas or Methylophaga.

2. A process as claimed in Claim 1 wherein the bacterium is Achromobacter methanophila, Methylobacillus glycogenes, Methylomonas methanolica, Methylomonas thalassica, Methylomonas clara, Methanomonas methylovora, Methanomonas methylovora subsp. thiaminophila, Protaminobacter candidus or Protaminobacter thiaminophagus, Methylobacterium organophilum, Protomonas extorquens, Mycoplana rubra, Ancylobacter aquaticus, Microcyclus eburneus, Microcyclus polymorphum, Microcyclus methanolica, Hyphomicrobium variable, Hyphomicrobium vulgare, Hyphomicrobium methylovorum, Hyphomicrobium sp., Xanthobacter autotrophicus, Xanthobacter flavus, Thiobacillus novellus, Thiobacillus versutus, Alteromonas thalassomethanolica, Methylophaga marina, or Methylophaga thalassica.

3. A process as claimed in Claim 1, wherein the bacterium is Achromobacter methanophila ATCC 21275, ATCC 21452 or ATCC 21961; Methylobacillus glycogenes ATCC 29475; Methylomonas methanolica NRRL B-5458; Methylomonas thalassica ATCC 33146; Methylomonas clara ATCC 31226; Methanomonas methylovora ATCC 21852, ATCC 21369, ATCC 21958 or ATCC 21963; Methanomonas methylovora subsp. thiaminophila ATCC 21370; Protaminobacter candidus ATCC 21372 or ATCC 21959; Protaminobacter thiaminophagus ATCC 21371, ATCC 21926, ATCC 21927, ATCC 21957 or ATCC 21969; Methylobacterium organophilum ATCC 29983; Protomonas extorquens JCM 2802, JCM 2805, JCM 2806, JCM 2811, JCM 2812, JCM 2813, JCM 2814, JCM 2815, JCM 2816, JCM 2817, JCM 2818, JCM 2819, JCM 2820, JCM 2821, JCM 2822, JCM 2823, JCM 2824, JCM 2825, JCM 2826, JCM 2827, JCM 2829, JCM 2830, JCM 2831, or JCM 2832; Mycoplana rubra NCIB 10409; Ancylobacter aquaticus ATCC 25396, DSM 334, ATCC 27068 or ATCC 27069; Microcyclus eburneus ATCC 21373; Microcyclus polymorphum NCIB 10516; Microcyclus methanolica DSM 2666, DSM 2667, DSM 2668 or DSM 2669; Hyphomicrobium variable NCIB 10517; Hyphomicrobium vulgare NCIB 9698 or NCIB 9775; Hyphomicrobium methylovorum IFO 14180; Hyphomicrobium sp. DSM 1869; Xanthobacter autotrophicus DSM 432, DSM 431, DSM 597, DSM 685, DSM 1393, DSM 1618, or DSM 2009; Xanthobacter flavus NCIB 10071; Thiobacillus novellus NCIB 10456 or ATCC 8093; or Thiobacillus versutus ATCC 25364; Alteromonas thalassomethanolica ATCC 33145; Methylophaga marina NCMB 2244, or

Methylophaga thalassica NCMB 2162 or NCMB 2163.

4. A process for the preparation of pyrrolo-quinoline quinone which comprises culturing a bacterium as defined below in a medium containing as a carbon source a source of methylamine and recovering pyrrolo-quinoline quinone thus produced from a culture broth or a supernatant of the culture broth, wherein the bacterium possesses the capability of utilizing methylamine to produce pyrrolo-quinoline quinone and belongs to the species Pseudomonas aminovorans.

5. A process as claimed in Claim 4, wherein the bacterium is Pseudomonas aminovorans NCIB 9039.

6. A process as claimed in any one of Claims 1 to 3, wherein the concentration of methanol in the culture broth is not more than 3 wt.%.

7. A process as claimed in any one of Claims 1 to 3, wherein the concentration of methanol in the culture broth is not more than 1.5 wt.%.

8. A process as claimed in any one of Claims 1 to 7, wherein the concentration of methylamine in the culture broth is not more than 1 wt.%.

9. A process as claimed in any one of Claims 1 to 7, wherein the concentration of methylamine in the culture broth is not more than 0.5 wt.%.

**Patentansprüche**
**Patentansprüche für folgende Vertragsstaaten : DE, FR, GB**

1. Verfahren zur Herstellung eines Pyrrolochinolinchinons, dadurch **gekennzeichnet,** daß eins der im folgenden definierten Bakterien in einem Medium, das als Kohlenstoffquelle eine Methanolquelle und/oder eine Methylaminquelle enthält, gezüchtet wird, und das so gezüchtete Pyrrolochinolinchinon aus der Kulturflüssigkeit oder dem Überstand der Kulturflüssigkeit gewonnen wird, wobei das Bakterium die Fähigkeit besitzt, Methanol und/oder Methylamin unter Herstellung von Pyrrolochinolinchinon zu verwenden und zum Genus Achromobacter, Methylobacillus, Methylomonas, Methanomonas, Protaminobacter (mit der Maßgabe, daß dies nur P. candidus und P. thiaminophagus umfaßt), Methylobacterium, Protomonas, Mycoplana, Ancylobacter, Microcyclus, Hyphomicrobium, Xanthobacter, Thiobacillus, Alteromonas oder Methylophaga gehört.

2. Verfahren nach Anspruch 1, dadurch **gekennzeichnet,** daß das Bakterium Achromobacter methanophila Methylobacillus glycogenes, Methylomonas methanolica, Methylomonas thalassica, Methylomonas clara, Methanomonas methylovora, Methanomonas methylovora subsp. thiaminophila, Protaminobacter candidus or Protaminobacter thiaminophagus, Methylobacterium organophilum, Protomonas extorquens, Mycoplana rubra, Ancylobacter aquaticus, Microcyclus eburneus, Microcyclus polymorphum, Microcyclus methanolica, Hyphomicrobium variable, Hyphomicrobium vulgare, Hyphomicrobium methylovorum, Hyphomicrobium sp., Xanthobacter autotrophicus, Xanthobacter flavus, Thiobacillus novellus, Thiobacillus versutus, Alteromonas thalassomethanolica, Methylophaga marina, oder Methylophaga thalassica ist.

3. Verfahren nach Anspruch 1, dadurch **gekennzeichnet,** daß das Bakterium Achromobacter methanophila ATCC 21275, ATCC 21452 oder ATCC 21961; Methylobacillus glycogenes ATCC 29475; Methylomonas methanolica NRRL B-5458; Methylomonas thalassica ATCC 33146; Methylomonas clara ATCC 31226; Methanomonas methylovora ATCC 21852, ATCC 21369, ATCC 21958 oder ATCC 21963; Methanomonas methylovora subsp. thiaminophila ATCC 21370; Protaminobacter candidus ATCC 21372 oder ATCC 21959; Protaminobacter thiaminophagus ATCC 21371, ATCC 21926, ATCC 21927, ATCC 21957 oder ATCC 21969; Methylobacterium organophilum ATCC 29983; Protomonas extorquens JCM 2802, JCM 2805, JCM 2806, JCM 2811, JCM 2812, JCM 2813, JCM 2814, JCM 2815, JCM 2816, JCM 2817, JCM 2818, JCM 2819, JCM 2820, JCM 2821, JCM 2822, JCM 2823, JCM 2824, JCM 2825, JCM 2826, JCM 2827, JCM 2829, JCM 2830, JCM 2831, oder JCM 2832; Mycoplana rubra NCIB 10409; Ancylobacter aquaticus ATCC 25396, DSM 334, ATCC 27068 oder ATCC 27069; Mi-crocyclus eburneus ATCC 21373; Microcyclus polymorphum NCIB 10516; Microcyclus methanolica DSM 2666, DSM 2667, DSM 2668 oder DSM 2669; Hyphomicrobium variable NCIB 10517; Hyphomicro-bium

vulgare NCIB 9698 oder NCIB 9775; Hyphomicrobium methylovorum IFO 14180; Hyphomicrobium sp. DSM 1869; Xanthobacter autotrophicus DSM 432, DSM 431, DSM 597, DSM 685, DSM 1393, DSM 1618, oder DSM 2009; Xanthobacter flavus NCIB 10071; Thiobacillus novellus NCIB 10456 oder ATCC 8093; oder Thiobacillus versutus ATCC 25364; Alteromonas thalassomethanolica ATCC 33145; Methylophaga marina NCMB 2244, oder Methylophaga thalassica NCMB 2162 oder NCMB 2163 ist.

**4.** Verfahren zur Herstellung eines Pyrrolochinolinchinons, dadurch **gekennzeichnet,** daß ein Bakterium wie es im folgenden definiert wird, in einem Medium, das als Kohlenstoffquelle eine Quelle für Methylamin enthält, gezüchtet wird, und das so gebildete Pyrrolochinolinchinon aus der Kulturflüssigkeit oder dem Überstand der Kulturflüssigkeit gewonnen wird, wobei das Bakterium die Fähigkeit besitzt, Methylamin unter Bildung eines Pyrrolochinolinchinons zu verwenden und zur Spezies Pseudomonas aminovorans gehört.

**5.** Verfahren nach Anspruch 4, dadurch **gekennzeichnet,** daß das Bakterium Pseudomonas aminovorans NCIB 9039 ist.

**6.** Verfahren nach einem der Ansprüche 1 bis 3, dadurch **gekennzeichnet,** daß die Konzentration von Methanol in der Kulturflüssigkeit nicht mehr als 3 Gew.-% beträgt.

**7.** Verfahren nach einem der Ansprüche 1 bis 3, dadurch **gekennzeichnet,** daß die Konzentration von Methanol in der Kulturflüssigkeit nicht mehr als 1,5 Gew.-% beträgt.

**8.** Verfahren nach einem der Ansprüche 1 bis 7, dadurch **gekennzeichnet,** daß die Konzentration an Methylamin in der Kulturflüssigkeit nicht mehr als 1 Gew.-% beträgt.

**9.** Verfahren nach einem der Ansprüche 1 bis 7, dadurch **gekennzeichnet,** daß die Konzentration an Methylamin in der Kulturflüssigkeit nicht mehr als 0,5 Gew.-% beträgt.

**Patentansprüche für folgenden Vertragsstaat : NL**

**1.** Verfahren zur Herstellung eines Pyrrolochinolinchinons, dadurch **gekennzeichnet,** daß eins der im folgenden definierten Bakterien in einem Medium, das als Kohlenstoffquelle eine Methanolquelle und/oder eine Methylaminquelle enthält, gezüchtet wird und das so gebildete Pyrrolochinolinchinon aus der Kulturflüssigkeit oder dem Überstand der Kulturflüssigkeit gewonnen wird, wobei das Bakterium die Fähigkeit besitzt Methanol und/oder Methylamin unter Herstellung von Pyrrolochinolinchinon zu verwenden und zum Genus Achromobacter, Methylobacillus, Methylomonas, Methanomonas, Protaminobacter, Methylobacterium, Protomonas, Mycoplana, Ancylobacter, Microcyclus, Hyphomicrobium, Xanthobacter, Thiobacillus, Alteromonas oder Methylophaga gehört.

**2.** Verfahren nach Anspruch 1, dadurch **gekennzeichnet,** daß das Bakterium Achromobacter methanophila Methylobacillus glycogenes, Methylomonas methanolica, Methylomonas thalassica, Methylomonas clara, Methanomonas methylovora, Methanomonas methylovora subsp. thiaminophila, Protaminobacter candidus or Protaminobacter thiaminophagus, Methylobacterium organophilum, Protomonas extorquens, Mycoplana rubra, Ancylobacter aquaticus, Microcyclus eburneus, Microcyclus Polymorphum, Microcyclus methanolica, Hyphomicrob ium variable, Hyphomicrobium vulgare, Hyphomicrobium methylovorum, Hyphomicrobium sp., Xanthobacter autotrophicus, Xanthobacter flavus, Thiobacillus novellus, Thiobacillus versutus, Alteromonas thalassomethanolica, Methylophaga marina, oder Methylophaga thalassica ist.

**3.** Verfahren nach Anspruch 1, dadurch **gekennzeichnet,** daß das Bakterium Achromobacter methanophila ATCC 21275, ATCC 21452 oder ATCC 21961; Methylobacillus glycogenes ATCC 29475; Methylomonas methanolica NRRL B-5458; Methylomonas thalassica ATCC 33146; Methylomonas clara ATCC 31226; Methanomonas methylovora ATCC 21852, ATCC 21369, ATCC 21958 oder ATCC 21963; Methanomonas methylovora subsp. thiaminophila ATCC 21370; Protaminobacter candidus ATCC 21372 oder ATCC 21959; Protaminobacter thiaminophagus ATCC 21371, ATCC 21926, ATCC 21927, ATCC 21957 oder ATCC 21969; Methylobacterium organophilum ATCC 29983; Protomonas extorquens JCM 2802, JCM 2805, JCM 2806, JCM 2811, JCM 2812, JCM 2813, JCM 2814, JCM 2815, JCM 2816, JCM 2817, JCM 2818, JCM 2819, JCM 2820, JCM 2821, JCM 2822, JCM 2823, JCM 2824, JCM 2825, JCM

2826, JCM 2827, JCM 2829, JCM 2830, JCM 2831, oder JCM 2832; Mycoplana rubra NCIB 10409; Ancylobacter aquaticus ATCC 25396, DSM 334, ATCC 27068 oder ATCC 27069; Mi-crocyclus eburneus ATCC 21373; Microcyclus polymorphum NCIB 10516; Microcyclus methanolica DSM 2666, DSM 2667, DSM 2668 oder DSM 2669; Hyphomicrobium variable NCIB 10517; Hyphomicro-bium vulgare NCIB 9698 oder NCIB 9775; Hyphomicrobium methylovorum IFO 14180; Hyphomicrobium sp. DSM 1869; Xanthobacter autotrophicus DSM 432, DSM 431, DSM 597, DSM 685, DSM 1393, DSM 1618, oder DSM 2009; Xanthobacter flavus NCIB 10071; Thiobacillus novellus NCIB 10456 oder ATCC 8093; oder Thiobacillus versutus ATCC 25364; Alteromonas thalassomethanolica ATCC 33145; Methylophaga marina NCMB 2244, oder Methylophaga thalassica NCMB 2162 oder NCMB 2163 ist.

**4.** Verfahren zur Herstellung eines Pyrrolochinolinchinons, dadurch **gekennzeichnet,** daß ein Bakterium wie es im folgenden definiert wird, in einem Medium, das als Kohlenstoffquelle eine Quelle für Methylamin enthält, gezüchtet wird, und das so gebildete Pyrrolochinolinchinon aus der Kulturflüssigkeit oder dem Überstand der Kulturflüssigkeit gewonnen wird, wobei das Bakterium die Fähigkeit besitzt, Methylamin unter Bildung eines Pyrrolochinolinchinons zu verwenden und zur Spezies Pseudomonas aminovorans gehört.

**5.** Verfahren nach Anspruch 4, dadurch **gekennzeichnet,** daß das Bakterium Pseudomonas aminovorans NCIB 9039 ist.

**6.** Verfahren nach einem der Ansprüche 1 bis 3, dadurch **gekennzeichnet,** daß die Konzentration von Methanol in der Kulturflüssigkeit nicht mehr als 3 Gew.-% beträgt.

**7.** Verfahren nach einem der Ansprüche 1 bis 3, dadurch **gekennzeichnet,** daß die Konzentration von Methanol in der Kulturflüssigkeit nicht mehr als 1,5 Gew.-% beträgt.

**8.** Verfahren nach einem der Ansprüche 1 bis 7, dadurch **gekennzeichnet,** daß die Konzentration an Methylamin in der Kulturflüssigkeit nicht mehr als 1 Gew.-% beträgt.

**9.** Verfahren nach einem der Ansprüche 1 bis 7, dadurch **gekennzeichnet,** daß die Konzentration an Methylamin in der Kulturflüssigkeit nicht mehr als 0,5 Gew.-% beträgt.

**Revendications**
**Revendications pour les Etats contractants suivants : DE, FR, GB**

**1.** Procédé de préparation de la pyrrolo-quinoléine quinone, qui consiste à cultiver une bactérie telle que définie ci-dessous dans un milieu contenant, comme source de carbone, une source de méthanol et/ou une source de méthylamine, et à recueillir la pyrrolo-quinoléine quinone ainsi produite dans un bouillon de culture ou dans une couche surnageante du bouillon de culture, la bactérie possédant l'aptitude à utiliser du méthanol et/ou de la méthylamine, pour produire de la pyrrolo-quinoléine quinone et appartenant au genre Achromobacter, Methylobacillus, Methylomonas, Methanomonas, Protaminobacter (pourvu que celui-ci englobe seulement P. candidus et P. thiaminonhagus), Methylobacterium, Protomonas, Mycoplana, Ancylobacter, Microcyclus, Hyphomicrobium, Xanthobacter, Thiobacillus, Alteromonas, ou Methylophaga.

**2.** Procédé suivant la revendication 1, dans lequel la bactérie est Achromobacter methanophila, Methylobacillus glycogenes, Methylomonas methanolica, Methylomonas thalassica, Methylomonas clara, Methanomonas methylovora, subsp thiaminophila, Protaminobacter candidus, ou Protaminobacter thiaminonhagus, Methylobacterium organophilum, Protomonas extorquens, Mycoplana rubra, Ancylobacter aquaticus, Microcyclus eburneus, Microcyclus polymorphum, Microcyclus methanolica, Hyphomicrobium variable, Hyphomicrobium vulgare, Hyphomicrobium methylovorum, Hyphomicrobium sp, Xanthobacter autotrophicus, Xanthobacter flavus, Thiobacillus novellus, Thiobacillus versutus, Alteromonas thalassomethanolica, Methylophaga marina, ou Methylophaga thalassica.

**3.** Procédé suivant la revendication 1, dans lequel la bactérie est Achromobacter methanophila ATCC 21275, ATCC 21275, ATCC 21452 ou ATCC 21961 ; Methylobacillus glycogenes, ATCC 29475 ; Methylomonas methanolica NRRL B-5458 ; Methylomonas thalassica ATCC 33146 ; Methylomonas clara ATCC 31226 ; Methanomonas methylovora ATCC 21852, ATCC 21369, ATCC 21958 ou ATCC

21963 ; Methanomonas methylovora subsp thiaminophila ATCC 21370 ; Protaminobacter candidus ATTC 21372 ou ATCC 21959 ; Protaminobacter thiaminophagus ATTC 21371, ATCC 21926, ATCC 21927, ATCC 21957 ou ATCC 21969 ; Methylobacterium organophilum ATCC 29983 ; Protomonas extorquens JCM 2802, JCM 2805, JCM 2806, JCM 2811, JCM 2812, JCM 2813, JCM 2814, JCM 2815, JCM 2816, JCM 2817, JCM 2818, JCM 2819, JCM 2820, JCM 2821, JCM 2822, JCM 2823, JCM 2824, JCM 2825, JCM 2826, JCM 2827, JCM 2829, JCM 2830, JCM 2831, ou JCM 2832 ; Mycoplana rubra NCIB 10409 ; Ancylobacter aquaticus ATCC 25396, DSM 334, ATCC 27068 ou ATCC 27069 ; Microcyclus eburneus ATCC 21373 ; Microcyclus polymorphum NCIB 10516 ; Microcyclus methanolica DSM 2666, DSM 2667, DSM 2668 ou DSM 2669 ; Hyphomicrobium variable NCIB 10517 ; Hyphomicrobium vulgare NCIB 9698 ou 9775 ; Hyphomicrobium methylovorum IFO 14180 ; Hyphomicrobium sp. DSM 1869 ; Xanthobacter autotrophicus DSM 432, DSM 431, DSM 597, DSM 685, DSM 1393, DSM 1618, ou DSM 2009 ; Xanthobacter flavus NCIB 10071 ; Thiobacillus novellus NCIB 10456 ou ATCC 8093 ; ou Thiobacillus versutus ATCC 25364 ; Alteromonas thalassomethanolica ATCC 33145 ; Methylophaga marina NCMB 2244, ou Methylophaga thalassica NCMB 2162 ou 2163.

**4.** Procédé de préparation de la pyrrolo-quinoléine quinone, qui consiste à cultiver une bactérie telle que définie ci-dessous dans un milieu contenant, comme source de carbone, une source de méthylamine, et à recueillir la pyrrolo-quinoléine quinone ainsi produite dans un bouillon de culture ou dans une couche surnageante du bouillon de culture, la bactérie possédant l'aptitude à utiliser la méthylamine pour produire de la pyrrolo-quinoléine quinone et appartenant à l'espèce Pseudomonas aminovorans.

**5.** Procédé suivant la revendication 4, dans lequel la bactérie est Pseudomonas aminovorans NCIB 9039.

**6.** Procédé suivant l'une quelconque des revendications 1 à 3, dans lequel la concentration de méthanol dans le bouillon de culture n'est pas supérieure à 3 % en poids.

**7.** Procédé suivant l'une quelconque des revendications 1 à 3, dans lequel la concentration de méthanol dans le bouillon de culture n'est pas supérieure à 1,5 % en poids.

**8.** Procédé suivant l'une quelconque des revendications 1 à 7, dans lequel la concentration de méthylamine dans le bouillon de culture n'est pas supérieure à 1 % en poids.

**9.** Procédé suivant l'une quelconque des revendications 1 à 7, dans lequel la concentration de méthylamine dans le bouillon de culture n'est pas supérieure à 0,5 % en poids.

**Revendications pour l'Etat contractant suivant : NL**

**1.** Procédé de préparation de la pyrrolo-quinoléine quinone, qui consiste à cultiver une bactérie telle que définie ci-dessous dans un milieu contenant, comme source de carbone, une source de méthanol et/ou une source de méthylamine, et à recueillir la pyrrolo-quinoléine quinone ainsi produite dans un bouillon de culture ou dans une couche surnageante du bouillon de culture, la bactérie possédant l'aptitude à utiliser du méthanol et/ou de la méthylamine, pour produire de la pyrrolo-quinoléine quinone et appartenant au genre Achromobacter, Methylobacillus, Methylomonas, Methanomonas, Protaminobacter, Methylobacterium, Protomonas, Mycoplana, Ancylobacter, Microcyclus, Hyphomicrobium, Xanthobacter, Thiobacillus, Alteromonas, ou Methylophaga.

**2.** Procédé suivant la revendication 1, dans lequel la bactérie est Achromobacter methanophila, Methylobacillus glycogenes, Methylomonas methanolica, Methylomonas thalassica, Methylomonas clara, Methanomonas methylovora, subsp thiaminophila, Protaminobacter candidus, ou Protaminobacter thiaminophagus, Methylobacterium organophilum, Protomonas extorquens, Mycoplana rubra, Ancylobacter aquaticus, Microcyclus eburneus, Microcyclus polymorphum, Microcyclus methanolica, Hyphomicrobium variable, Hyphomicrobium vulgare, Hyphomicrobium methylovorum, Hyphomicrobium sp, Xanthobacter autotrophicus, Xanthobacter flavus, Thiobacillus novellus, Thiobacillus versutus, Alteromonas thalassomethanolica, Methylophaga marina, ou Methylophaga thalassica.

**3.** Procédé suivant la revendication 1, dans lequel la bactérie est Achromobacter methanophila ATCC 21275, ATCC 21275, ATCC 21452 ou ATCC 21961 ; Methylobacillus glycogenes, ATCC 29475 ; Methylomonas methanolica NRRL B-5458 ; Methylomonas thalassica ATCC 33146 ; Methylomonas

clara ATCC 31226 ; Methanomonas methylovora ATCC 21852, ATCC 21369, ATCC 21958 ou ATCC 21963 ; Methanomonas methylovora subsp thiaminophila ATCC 21370 ; Protaminobacter candidus ATTC 21372 ou ATCC 21959 ; Protaminobacter thiaminophagus ATTC 21371, ATCC 21926, ATCC 21927, ATCC 21957 ou ATCC 21969 ; Methylobacterium organophilum ATCC 29983 ; Protomonas extorquens JCM 2802, JCM 2805, JCM 2806, JCM 2811, JCM 2812, JCM 2813, JCM 2814, JCM 2815, JCM 2816, JCM 2817, JCM 2818, JCM 2819, JCM 2820, JCM 2821, JCM 2822, JCM 2823, JCM 2824, JCM 2825, JCM 2826, JCM 2827, JCM 2829, JCM 2830, JCM 2831, ou JCM 2832 ; Mycoplana rubra NCIB 10409 ; Ancylobacter aquaticus ATCC 25396, DSM 334, ATCC 27068 ou ATCC 27069 ; Microcyclus eburneus ATCC 21373 ; Microcyclus polymorphum NCIB 10516 ; Microcyclus methanolica DSM 2666, DSM 2667, DSM 2668 ou DSM 2669 ; Hyphomicrobium variable NCIB 10517 ; Hyphomicrobium vulgare NCIB 9698 ou 9775 ; Hyphomicrobium methylovorum IFO 14180 ; Hyphomicrobium sp. DSM 1869 ; Xanthobacter autotrophicus DSM 432, DSM 431, DSM 597, DSM 685, DSM 1393, DSM 1618, ou DSM 2009 ; Xanthobacter flavus NCIB 10071 ; Thiobacillus novellus NCIB 10456 ou ATCC 8093 ; ou Thiobacillus versutus ATCC 25364 ; Alteromonas thalassomethanolica ATCC 33145 ; Methylophaga marina NCMB 2244, ou Methylophaga thalassica NCMB 2162 ou 2163.

**4.** Procédé de préparation de la pyrrolo-quinoléine quinone, qui consiste à cultiver une bactérie telle que définie ci-dessous dans un milieu contenant, comme source de carbone, une source de méthylamine, et à recueillir la pyrrolo-quinoléine quinone ainsi produite dans un bouillon de culture ou dans une couche surnageante du bouillon de culture, la bactérie possédant l'aptitude à utiliser la méthylamine pour produire de la pyrrolo-quinoléine quinone et appartenant à l'espèce Pseudomonas aminovorans.

**5.** Procédé suivant la revendication 4, dans lequel la bactérie est Pseudomonas aminovorans NCIB 9039.

**6.** Procédé suivant l'une quelconque des revendications 1 à 3, dans lequel la concentration de méthanol dans le bouillon de culture n'est pas supérieure à 3 % en poids.

**7.** Procédé suivant l'une quelconque des revendications 1 à 3, dans lequel la concentration de méthanol dans le bouillon de culture n'est pas supérieure à 1,5 % en poids.

**8.** Procédé suivant l'une quelconque des revendications 1 à 7, dans lequel la concentration de méthylamine dans le bouillon de culture n'est pas supérieure à 1 % en poids.

**9.** Procédé suivant l'une quelconque des revendications 1 à 7, dans lequel la concentration de méthylamine dans le bouillon de culture n'est pas supérieure à 0,5 % en poids.